## Europäisches Patentamt
## European Patent Office
## Office européen des brevets

(19)

(11) Veröffentlichungsnummer: **0 262 488**
**B1**

(12)

# EUROPÄISCHE PATENTSCHRIFT

(45) Veröffentlichungstag der Patentschrift:
06.06.90

(21) Anmeldenummer: 87113290.8

(22) Anmeldetag: 11.09.87

(51) Int. Cl.⁵: **C08F 2/44, C09C 3/08, A61K 6/08**

(54) Reaktiver organischer Füllstoff sowie dessen Verwendung.

(30) Priorität: 23.09.86 DE 3632215

(43) Veröffentlichungstag der Anmeldung:
06.04.88 Patentblatt 88/14

(45) Bekanntmachung des Hinweises auf die Patenterteilung:
06.06.90 Patentblatt 90/23

(84) Benannte Vertragsstaaten:
AT CH DE FR GB IT LI SE

(56) Entgegenhaltungen:
DE-A- 1 592 825
DE-A- 2 126 419

(73) Patentinhaber: Ivoclar AG, FL-9494 Schaan(LI)

(72) Erfinder: Rheinberger, Volker M., Dr., Floraweg 3,
FL-9490 Vaduz(LI)
Erfinder: Büchel, Thomas, Duxweg 14,
FL-9494 Schaan(LI)

(74) Vertreter: UEXKÜLL & STOLBERG Patentanwälte,
Beselerstrasse 4, D-2000 Hamburg 52(DE)

**Beschreibung**

Die Erfindung betrifft neuartige reaktive organische Füllstoffe in Form eines Feststoffpulvers für polymerisierbare Zusammensetzungen, welche als Bindemittel Verbindungen mit radikalisch polymerisierbaren, ethylenisch ungesättigten Gruppen enthalten. Solche Zusammensetzungen finden auf verschiedenen technischen Gebieten Anwendung; besondere Bedeutung haben sie als Dentalwerkstoffe erlangt. Unter Dentalwerkstoffen werden z.B. Zahnfüllmassen für die konservierende Zahnbehandlung und Materialien zur Herstellung von künstlichen Zähnen oder Zahnteilen, wie Kronen oder Inlays, verstanden.

Es ist bereits bekannt, in eine Matrix aus polymerisierbaren Monomeren verschiedenartige Füller einzuarbeiten. Einerseits soll dadurch eine Verdickung erreicht werden, welche zu einer Konsistenz der Zusammensetzung führt, die deren Handhabung vor der Polymerisation ermöglicht. Andererseits sollen durch den Füllerzusatz die Eigenschaften des auspolymerisierten Materials, insbesondere dessen Härte und Druckfestigkeit, verbessert und die Schrumpfneigung vermindert werden.

Weit verbreitet finden anorganische Füllstoffe unterschiedlicher chemischer Zusammensetzung Verwendung. Da anorganische Füllstoffe jedoch im allgemeinen nur einen schlechten Verbund mit einer organischen Matrix liefern, konnten die mechanischen Eigenschaften der Polymerisate zunächst nicht befriedigen. Zur Verbesserung des Verbundes werden die anorganischen Füllstoffe oberflächlich mit einem Silan behandelt. Auch solche silanisierten anorganischen Füllstoffe vermögen jedoch nicht alle gestellten Anforderungen voll zu befriedigen.

Es ist ferner bekannt, organische Perlpolymerisate, welche gegebenenfalls ihrerseits anorganische Füllstoffe enthalten können, zu verwenden. Solche Perlpolymerisate werden vorzugsweise aus denselben Monomeren hergestellt, welche auch als Bindemittel dienen, um eine möglichst große Verträglichkeit zu erreichen. Bekannt sind beispielsweise Perlpolymerisate aus Polymethylmethacrylat oder anderen radikalisch polymerisierten Acrylaten, vgl. DE-OS 28 50 918 und EP-OS 23 321. Die radikalische Polymerisation läßt sich nur schwierig steuern, und es bleibt stets ein Anteil an unreagierten Monomeren zurück. Auch werden solche Füllstoffe in der Matrix im allgemeinen nur ungenügend verankert. Füllstoffe mit einem mittleren Durchmesser von mehr als 1 µm verleihen einem Dentalwerkstoff die nachteilige Eigenschaft, daß er nach der Aushärtung nur ungenügend poliert werden kann, und daß bei längerer Kaubeanspruchung Füllstoffteilchen herausbrechen, was auch den Verschleiß des Matrixpolymeren beschleunigt. Diese Probleme werden sowohl bei silanisierten grobteiligen anorganischen Füllern als auch bei Perlpolymeren, welche mit silanisiertem anorganischen Füllern gefüllt sind, beobachtet.

Das Problem besteht somit darin, daß bei den bekannten anorganischen und organischen Füllstoffen der Verbund zwischen Füllstoff und Matrix zu schwach ist. Bisher ist es nicht gelungen, einen Füllstoff so zu modifizieren, daß der Verbund mit der auspolymerisierten Matrix wirklich in jeder Beziehung befriedigt. Alle größeren Abwandlungen in der Zusammensetzung oder Herstellung des Füllstoffes führten gleichzeitig zu einschneidenden Änderungen der physikalischen Eigenschaften, so daß ein Einsatz als fester Füllstoff nicht mehr in Frage kommt.

Der Erfindung liegt die Aufgabe zugrunde, einen organischen Füllstoff zu entwickeln, der durch einen hohen Gehalt an reaktiven, mit den Matrixmonomeren copolymerisierbaren Gruppen einen festen Verbund zwischen Füllstoff und Matrix liefert und damit nach dem Auspolymerisieren Werkstoffe ergibt, die eine wesentlich verbesserte Rißfestigkeit, Belastbarkeit und Abriebfestigkeit aufweisen.

Gegenstand der Erfindung ist ein reaktiver organischer Füllstoff in Form eines Feststoffpulvers, welcher dadurch gekennzeichnet ist, daß er durch eine nicht radikalisch ausgelöste Reaktion hergestellt ist und je Gramm des organischen Füllstoffes mindestens 0,5 mMol mit Lösungsmitteln nicht extrahierbare, reaktive Doppelbindungen (bestimmt mittels der DSC-Methode) aufweist.

Bei Einsatz des erfindungsgemäßen Füllstoffes in polymerisierbaren Zusammensetzungen, welche als Bindemittel Verbindungen mit radikalisch polymerisierbaren, ethylenisch ungesättigten Gruppen enthalten, führt der relativ hohe Gehalt des Füllstoffes an reaktiven Doppelbindungen zu einem bislang nicht erreichten festen Verbund zwischen Füllstoff und Matrix, wie eine Untersuchung der auspolymerisierten Werkstoffe zeigt: Im Ermüdungstest überstehen sie ein Vielfaches der Belastungszyklen herkömmlicher Werkstoffe, welche spätestens nach zehn Zyklen eine Rißbildung zeigen. Darüber hinaus ergibt die elektronenmikroskopische Untersuchung (REM) der Rißbilder, daß bei herkömmlichen Werkstoffen die Risse längs der Grenzlinien zwischen Matrix und Füllstoffteilchen entstehen, während bei den erfindungsgemäß hergestellten Werkstoffen der Verbund zwischen Füllstoff und Matrix so fest ist, daß die Risse geradlinig entlang der Eindruckstelle, also durch Matrix und Füllstoffteilchen hindurch, verlaufen.

Vorzugsweise liegt der Gehalt des erfindungsgemäßen Füllstoffes an Doppelbindungen bei 0,5 bis 5,0, insbesondere bei 1,0 bis 3,0 und besonders bevorzugt bei 1,4 bis 2,6 mMol/g des organischen Füllstoffes. Dieser hohe Gehalt an reaktiven Doppelbindungen wird dadurch erreicht, daß der Füllstoff durch eine nicht radikalisch ausgelöste Reaktion, vorzugsweise eine Additionsreaktion, polymerisiert wird, so daß 75 bis 90% der in den eingesetzten Monomeren enthaltenen ethylenischen Doppelbindungen auch noch in dem Füllstoffpulver vorhanden sind. Bei der herkömmlichen Herstellung von Perlpolymerisaten mittels radikalischer Polymerisation werden dagegen die meisten Doppelbindungen durch die Polymerisationsreaktion verbraucht. Da erfindungsgemäß die Monomeren im stöchiometrischen Verhältnis miteinander umgesetzt werden, enthält der Füllstoff praktisch keine unumgesetzten Monomeren, was sich daran zeigt, daß der Gehalt an Doppelbindungen durch Lösungsmittelextraktion nicht vermindert werden kann. Die Füllstoffe sind dadurch härter als solche, die noch Monomere enthalten.

Die im organischen Füllstoff enthaltenen reaktiven Doppelbindungen lassen sich mittels der DSC-Methode (Differential Scanning Calorimetry) quantitativ bestimmen. Dazu wird eine genau eingewogene Probe des Füllstoffes mit einer bestimmten Menge einer standardisierten Peroxidlösung versetzt, worauf das Lösungsmittel vorsichtig abgedampft wird. Eine genau abgewogene Menge des mit Peroxid beschichteten Füllstoffes wird in einem Perkin-Elmer®-Gerät Typ DSC-2 erhitzt, und die bei der stattfindenden exothermen Reaktion freiwerdende Wärmemenge wird aus dem Thermogramm ermitteln. Durch Vergleich mit den Werten für die Polymerisationswärme der eingesetzten Monomeren läßt sich der Polymerisationsgrad exakt bestimmen. Der vorliegend verwendete Ausdruck "reaktive Doppelbindungen des Füllstoffes" ist in diesem Sinne gemeint.

Vorzugsweise ist der erfindungsgemäße Füllstoff durch Umsetzung von Hydroxygruppen enthaltenden (Meth)acrylaten mit Isocyanaten im Verhältnis von OH-Gruppen zu NCO-Gruppen von etwa 1:1 hergestellt, wobei mindestens eine der Ausgangsverbindungen tri- oder höherfunktionell ist, um den für die Gewinnung eines Feststoffpulvers ausreichenden Vernetzungsgrad zu erreichen. Gemäß einer besonders günstigen Ausführungsform geht man von einem Tri- oder Polyisocyanat aus, wobei es dann auch möglich ist, einen stöchiometrischen Unterschuß an Hydroxy(meth)acrylat einzusetzen und die erforderliche Vernetzung mit Wasser und/oder einem Polyol, z.B. einem aliphatischen Triol, zu erreichen, welche mit den nicht umgesetzten Isocyanatgruppen unter Harnstoff- bzw. Urethangruppenbildung reagieren. Andererseits kann man auch ein (Meth)acrylat mit drei oder mehr Hydroxygruppen mit einem Diisocyanat umsetzen.

Beispiele für geeignete hydroxyfunktionelle (Meth)acrylate sind Hydroxyethylmethacrylat (HEMA), Polyethylenglycolmethacrylat, 2-Hydroxy- und 2,3-Dihydroxypropylmethacrylat, Pentaerythritoltriacrylat sowie Raktionsprodukte von Glycidyl(meth)acrylat mit Polyolen, z.B. Trimethylolpropan, oder Polycarbonsäuren, z.B. Bernsteinsäure. Besonders bevorzugt ist Bis-GMA (Bisphenol A-glycidylmethacrylat).

Bevorzugte Isocyanate sind aliphatische Verbindungen, wie 3-Isocyanatomethyl-3,5,5-trimethylcyclohexylisocyanat, Trimethylhexamethylendiisocyanat sowie das Triisocyanat Tris(6-isocyanatohexyl)biuret (Desmodur® N 100 der Bayer AG).

Die Umsetzung zwischen denHydroxy(meth)acrylaten und Isocyanaten läßt sich unter milden Bedingungen durchführen. Die Temperatur beträgt maximal etwa 150°C, vorzugsweise etwa 10 bis 60°C. Zur Beschleunigung kann ein Katalysator zugesetzt werden; geeignet sind u.a. tertiäre Amine und metallorganische Salze.

Die Umsetzung von hydroxyfunktionellen (Meth)acrylaten ist als solche bereits bekannt. Sie dient beispielsweise zur Herstellung von Präpolymeren, welche als Bindemittel in Dentalwerkstoffen Verwendung finden können, vergl. DE-OS 2 126 419.

Der erfindungsgemäße Füllstoff läßt sich auch durch ionische Copolymerisation von Hydroxy(meth)acrylaten mit einem Epoxyharz und/oder Trioxan im stöchiometrischen Verhältnis herstellen. Beispielsweise kann Bisphenol A-diglycidylether (Epikote® 828) mit Glycidylmethacrylat und/oder HEMA unter Verwendung von BF₃ als Katalysator umgesetzt werden. Ähnliche Ergebnisse liefert die Umsetzung von Glycidyl(meth)acrylat mit Trioxan und von Epoxiden mit Epoxy(meth)acrylaten. Weitere Beispiele sind die Umsetzung von Hydroxyverbindungen mit Carbonsäurederivaten, wobei mindestens eine der Ausgangsverbindungen (Meth)acrylatgruppen enthält, zu Polyestern, und die Reaktion von Allyliden, z.B. von Diallyliden-pentaerythritol, mit Alkoholen oder Carbonsäuren. Auch bei diesen Umsetzungen bleiben die ethylenisch ungesättigten Vinylgruppen unverändert und stehen als reaktive Gruppen für die spätere Reaktion mit dem Matrixbindemittel zur Verfügung.

Der erfindungsgemäße Füllstoff wird nach seiner Herstellung durch Mahlen auf die erforderliche Teilchengröße gebracht. Vorzugsweise liegt die mittlere Teilchengröße des gemahlenen Füllstoffes im Bereich von 0,5 bis 100 μm. Besonders günstig ist eine mittlere Teilchengröße von etwa 10 bis 50 μm.

Gemäß einer vorteilhaften Ausführungsform der Erfindung wird in den reaktiven organischen Füllstoff ein anorganischer und/oder nicht reaktiver organischer Füllstoff inkorporiert, was vorteilhafterweise dadurch geschieht, daß der Füllstoff den Ausgangsverbindungen vor der Additionsreaktion zugesetzt wird. Die physikalischen Eigenschaften des Füllstoffes lassen sich dadurch zusätzlich in weiten Grenzen variieren. Der Gehalt an inerten Füllstoffen im reaktiven Füllstoff kann, bezogen auf das Gesamtgewicht des Füllstoffes - zwischen 0 und 80 Gew.% liegen. Besonders günstig sind Gehalte von 20 bis 75, insbesondere von etwa 40 bis 70 Gew.%.

Eine große Zahl von anorganischen Verbindungen ist als Füllstoff geeignet. Beispiele sind Glaspulver, Aluminiumoxid, Siliciumdioxid wie Quarz, Sand oder Kieselsäure, Diatomeenerde, Calciumcarbonat, Ton, Talk, Bimsstein, Schlackenmehl, Glimmer, Asbest, Aluminiumsulfat, Calciumsulfat oder Lithopone. Ebenfalls geeignet sind Molybdänsulfid, Graphit, Ruß, Flugasche, Kaliumtitanat oder auch Fasern, z.B. Glasfasern bzw. Kohlenstoffasern. Bei Verwendung des Füllstoffes in Dentalwerkstoffen sind Glaspulver bzw. Quarzpulver sowie feinstteilige Kieselsäuren, insbesondere mikrofeine pyrogene, aber auch gefällte Kieselsäuren, besonders geeignet. Eine weitere bevorzugte Gruppe sind anorganische Füllstoffe, welche den fertigen Dentalwerkstoff röntgenopak machen, wie Bariumsulfat oder Fluoride von Seltenen Erdmetallen.

3

Für viele Anwendungen wird der anorganische Füllstoff vorzugsweise oberflächlich silanisiert, um seine Einarbeitung in die organischen Materialien zu erleichtern und - bei Einsatz von Silanen mit polymerisierbaren Doppelbindungen - eine gewisse Bindung zwischen der organischen Matrix und dem anorganischen Füller zu erreichen. Ein besonders bevorzugtes Silan ist γ-Methacryloxypropyltrimethoxysilan. Geeignete Silane sind ferner solche mit Hydroxy-, Amino- und Epoxidgruppen.

Es ist wichtig, bei der Herstellung des erfindungsgemäßen Füllstoffes zu berücksichtigen, daß der gegebenenfalls zugefügte anorganische Füller oberflächlich Gruppen enthalten kann, welche an der Reaktion teilnehmen; beispielsweise weisen Kieselsäuren oberflächlich Silanolgruppen Si-OH auf, welche mit Isocyanatgruppen zu reagieren vermögen. Der Gehalt des anorganischen Füllers an solchen OH-Gruppen muß daher bei Einstellung des OH:NCO-Verhältnisses der Ausgangsverbindungen berücksichtigt werden.

Geeignete inerte organische Füllstoffe sind insbesondere Acryl- und Methacrylpolymere, z.B. Polymethylmethacrylat, und Polyurethane. Diese Polymerisate werden durch Vermahlen auf die gewünschte Teilchengröße gebracht.

Gegenstand der Erfindung ist ferner die Verwendung der vorstehend näher beschriebenen reaktiven organischen Füllstoffe in polymerisierbaren Zusammensetzungen, welche als Bindemittel Verbindungen mit radikalisch polymerisierbaren ethylenisch ungesättigten Gruppen enthalten. Geeignete Vinylverbindungen für solche Zusammensetzungen mit dem erfindungsgemäßen reaktiven organischen Füllstoff sind u.a. 4-Methacryloxyethyltrimellithsäure und deren Anhydrid, Epoxyacrylate des Bisphenol-Typs und deren Oligomere, Urethandimethacrylat, Methylacrylat, Methyl-, Ethyl- und Butylmethacrylat, Polyethylenglykoldimethacrylat, 2,2-Bis(p-2′-hydroxy3′-methacryloxypropoxyphenyl)-propan, 2,2-Bis-(4-methacryloxypolyethoxyphenyl)-propan, Acrylnitril, Vinylacetat, 2-Cyanoacrylsäure, Styrol, Divinylbenzol sowie Mischungen der vorstehenden Monomeren. Die Bindemittel können auch Vinylgruppen enthaltende Präpolymere oder Polymere sein.

Besonders geeignet sind die Füllstoffe zur Verwendung in radikalisch polymerisierbaren, insbesondere in lichthärtenden Dentalwerkstoffen, welche als Bindemittel ebenfalls Vinylverbindungen enthalten. Besonders eignen sich hierfür monofunktionelle oder polyfunktionelle Methacrylate, die allein oder in Mischungen eingesetzt werden können. Als Beispiele für diese Verbindungen kommen Methyl-, Isobutyl- und Cyclohexylmethacrylat, Triethylenglycoldimethacrylat, Diethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, aber auch Bis-GMA sowie die Reaktionsprodukte aus Isocyanaten, insbesondere Di- und/oder Triisocyanaten und OH-gruppenhaltigen Methacrylaten in Frage. Beispiele dafür sind die Umsetzungsprodukte von 1 Mol Hexamethylendiisocyanat mit 2 Mol 2-Hydroxyethylenmethacrylat, von 1 Mol Tris(6-isocyanatohexyl)biuret mit 3 Mol Hydroxyethylmethacrylat und von 1 Mol Trimethylhexamethylendiisocyanat mit 2 Mol Hydroxyethylmethacrylat, die kurz als Urethandimethacrylat bezeichnet werden können. Der Anteil dieser meist langkettigen Verbindungen im Dentalwerkstoff bewegt sich zwischen 10 und 50 Gew.%.

Zur näheren Erläuterung der Erfindung sollen die nachfolgenden Beispiele dienen, auf welche die Erfindung jedoch nicht beschränkt ist.

BEISPIEL 1

In einem Mörser wird eine Lösung aus 19 g Hydroxyethylmethacrylat, 2,7 g Wasser und 0,01 g Dibutylzinndiacetat vorgelegt und mit 20 g silanisiertem Siliciumdioxid mit einer mittleren Primärteilchengröße von 40 nm (AEROSIL® OX-50 der Fa. Degussa) versetzt. Zu dieser Mischung gibt man dann 86 g Tris(6-isocyanatohexyl)-biuret und mischt weitere 88 g des Siliciumdioxid-Füllstoffs möglichst homogen hinein. Auf einem Drei-Walzen-Stuhl wird die Masse völlig homogenisiert.

Die Aushärtung erfolgt in einem Wärmeschrank bei 50°C während 100 h. Der harte Kunststoff wird gebrochen, in einer Keramik-Kugelmühle gemahlen und durch ein Sieb mit der Maschenweite von 90 μm von größeren Anteilen befreit.

Die Löslichkeit bestimmt man in einer Soxhletapparatur während 24 h mit Aceton zu 0,9%. Nach der DSC-Methode ergibt sich ein Doppelbindungsgehalt von 1,6 mMol pro g organischer Substanz.

BEISPIEL 2

In einem Mörser werden 41 g Trimethylolpropanmonoglycidylmethacrylataddukt und 40 g des in Beispiel 1 verwendeten SiO₂ (AEROSIL® OX-50) zusammengemischt. Nach der Zugabe von 69 g Tris(6-isocyanatohexyl)-biuret mischt man weitere 70 g SiO₂ dazu und homogenisiert die entstandene Paste auf dem Drei-Walzen-Stuhl. Nach einer Lagerung von 140 h bei Raumtemperatur wird der entstandene Kunststoff gebrochen, in einer Keramik-Kugelmühle gemahlen und durch ein 90 μm-Sieb gesiebt.

Die Löslichkeit beträgt 2,0%. Mittels DSC können 1,4 mMol pro g organische Substanz Doppelbindungen nachgewiesen werden.

## BEISPIEL 3

Es wurde wie Beispiel 2 gearbeitet. Anstelle von Trimethylolpropanmonoglycidylmethacrylat werden 50 g Trimethylolpropandiglycidylmethacrylat eingesetzt. Dazu kommen 70 g Tris(6-isocyanatohexyl)-biuret und 118 g $SiO_2$ nach Beispiel 1 (AEROSIL® OX-50), das mit 10 $\gamma$-Methacryloxypropyltrimethoxysilan silanisiert wird.

Die Löslichkeit beträgt 1,1% und der Doppelbindungsanteil 1,7 mMol pro g organischer Substanz.

## BEISPIEL 4

Zur Abwandlung von Beispiel 2 werden 31 g 2,3-Di hydroxypropylmethacrylat, 69 g Tris(6-isocyanato-hexyl)-biuret und 100 g silanisierter Füllstoff nach Beispiel 1 (AEROSIL® OX-50) auf dem Drei-Walzen-Stuhl homogenisiert.

Die Löslichkeit beträgt 0,8% und der Doppelbindungsanteil 1,9 mMol pro g organischer Substanz.

## BEISPIEL 5

Zur Abwandlung von Beispiel 2 werden 75 g Bernsteinsäurediglycidylmethacrylat, 69 g Tris(6-isocya-natohexyl)-biuret und 144 g silanisiertes Siliciumdioxid (AEROSIL® OX-50) auf dem Drei-Walzen-Stuhl homogenisiert.

Die Löslichkeit beträgt 2,0% und der Doppelbindungsanteil 2,0 mMol pro g organischer Substanz.

## BEISPIEL 6

Alle 3 Komponenten, nämlich 89 g Tris(6-isocyanatohexyl)-biuret, 121 g silanisiertes Siliciumdioxid (AEROSIL® OX-50) und eine Mischung aus 19 g Hydroxyethylmethacrylat und 14 g Trimethylolpropan werden auf 50°C erwärmt und anschließend zu einer Paste geknetet. Zusammen mit einer Lösung aus 1 g Hydroxyethylmethacrylat und 0,12 g Dibutylzinndiacetat erfolgt auf dem Drei-Walzen-Stuhl eine vollständige Homogenisierung.

Die Löslichkeit beträgt 1,7% und der Doppelbindungsanteil 1,7 mMol pro g organischer Substanz.

## BEISPIEL 7

In die Labor-Universal-Knetmaschine LUK® 025 der Fa. Werner & Pfeidler (BRD) werden 41 g Bisphenol A-glycidylmethacrylat und 29 g Tris(6-isocyanatohexyl)-biuret gegeben und 130 g silanisiertes Siliciumdioxid (AEROSIL® OX-50) hineingeknetet. Die entstandene Paste läßt man bei 60°C während 80 h aushärten, bricht den Kunststoff und mahlt ihn in einer Keramik-Kugelmühle während 30 h. Nach dem Sieben durch ein 90 μm-Sieb erhält man ein Pulver mit einer durchschnittlichen Korngröße von 31 μm.

Die Löslichkeit in Aceton beträgt 1,8% und der Doppelbindungsanteil (mittels DSC) 2,6 mMol/g organischer Substanz.

## BEISPIEL 8

Zur Abwandlung von Beispiel 7 werden anstelle 65 % nur 40 % Siliciumdioxid eingesetzt. Das Verhältnis Bisphenol A-glycidylmethacrylat zu Tris(6-isocyanatohexyl)-biuret bleibt gleich.

Die Löslichkeit in Aceton beträgt 2,3 % und der Doppelbindungsanteil 2,2 mM pro g organischer Substanz.

## BEISPIEL 9

In einem Gefäß werden 59 g Bisphenol A-glycidylmethacrylat und 41 g Tris(6-isocyanatohexyl)-biuret durch Rühren homogen vermischt. Nach einer Härtezeit von 70 h bei 50°C wird der Füller gemahlen und durch ein 90 μm-Sieb gesiebt.

Die Korngröße mißt durchschnittlich 42,5 μm. Die Löslichkeit beträgt 2,0 % und der Doppelbindungsgehalt 1,7 mMol/g.

## BEISPIEL 10

In Abwandlung von Beispiel 7 werden 37 g silanisiertes Siliciumdioxid mit einer mittleren Primärteilchengröße von 16 nm und einer BET-Oberfläche von 110 ± 20 $m^2$/g (AEROSIL R972 der Fa. Nippon Aerosil Co., Ltd.) eingesetzt.

Der Gehalt an Doppelbindungen beträgt 2,1 mMol/g organische Substanz.

BEISPIEL 11

In einem Gefäß werden 100 g Trimethylolpropantriglycidylmethacrylat, 172 g 3-Isocyanatomethyl-3,5,5-trimethylcyclohexylisocyanat und 181 g silanisiertes Siliciumdioxid wie in Beispiel 1 homogen gemischt. Nach der Zugabe von 0,1 % Dibutylzinndiacetat härtet der Füller innerhalb von 70 h aus, wird gemahlen und gesiebt.

Der Doppelbindungsanteil beträgt 1,5 mMol/g organische Substanz.

BEISPIEL 12 ( Vergleichsbeispiel) (radikalisch polymerisierter Füller)

In einer Knetmaschine wird in eine Lösung aus 79 % G 2,2,4-Trimethylhexamethylen-bis(2-carbamoyloxyethyl)dimethacrylat, 20 % G Decandiol-1,10-dimethacrylat und 2 % G Benzoylperoxid eine gewichtsmäßig gleiche Menge silanisiertes Siliciumdioxid gemäß Beispiel 1 eingearbeitet. Die Masse wird während 24 h bei 120°C polymerisiert. Das Polymer wird anschließend zerschlagen und in einer Kugelmühle 60 h aufgemahlen. Die mittlere Korngröße des nach dem Sieben erhaltenen Füllers liegt zwischen 30 und 40 μm.

Der Doppelbindungsanteil beträgt < 0,5 mMol/g organische Substanz, die Löslichkeit 3,1 % G.

BEISPIEL 13

In den Laborkneter werden 52,6 g einer Monomerenmischung mit folgender Zusammensetzung vorgelegt:

84,21% 2,2,4-Trimethylhexamethylen-bis-(2-carbamoyloxyethyl)-dimethacrylat (RM-3)
15,00% Dekandioldimethacrylat (D3MA)
00,28% DL-Campherchinon
00,49% Cyanoethylmethylanilin (CEMA)
00,03% 2,6-Di-tert.-butyl-p-Kresol (BHT).

Dazu werden 122,8 g des erfindungsgemäßen Füllers gemäß Beispiel 7 und 24,4 g silanisiertes Siliciumdioxid (AEROSIL® OX-50) hineingeknetet, so daß eine stopfbare Paste entsteht.

Die Paste wird in eine Metallform (Abmessung Höhe 4 mm, Breite 4 mm und Länge 20 mm) gefüllt und von beiden Seiten 90 Sekunden lang mit einem Lichtpolymerisationsgerät belichtet. Die Prüfkörper, aus der Form entnommen, werden mit 1000er-Schleifpapier geschliffen, mit Aluminiumoxid poliert und eine Woche bei Raumtemperatur in Wasser gelagert.

Die Prüfkörper werden einem Ermüdungstest unterworfen. Als Prüfgerät wird eine Wolpert/Amsler-Werkstoffprüfmaschine Typ 2TZM 771 20 KN verwendet.

Beim Test wird eine Stahlkugel mit 2 mm Durchmesser auf die zur Auflagefläche planparallele Oberfläche gedrückt und wieder entlastet. Der Kraftbereich für einen solchen Zyklus liegt zwischen 300 N bei Belastung und 50 N bei Entlastung. Die Geschwindigkeit beträgt 100 mm/Min. Bestimmt wird diejenige Anzahl Zyklen, bei denen das Material schadlos bleibt, d.h. daß sich um den Eindruckrand keine Risse zeigen, die mit dem Lichtmikroskop bei 100facher Vergrößerung sichtbar sind.

Mit dem obenbeschriebenen Material treten Risse erst nach 5000 Zyklen auf. Das Rißbild zeigt einen geradlinigen Rißverlauf, was auf einen guten Verbund zwischen Füllkörnern und der Matrix schließen läßt, da der Bruch durch die Körner und nicht entlang der Körner geht. Diese homogene Bruchverhalten bestätigt, daß der erfindungsgemäß hergestellte Füller genügend Doppelbindungen enthält, um mit der Matrix einen festen Verbund einzugehen.

BEISPIEL 14

Das Monomerengemisch enthält abweichend von Beispiel 13 anstelle der 84,21% RM-3 15%, der Rest wird durch Bisphenol A-glycidylmethacrylat (Bis-GMA) ersetzt. Die Paste ist wie folgt zusammengesetzt:

24,36% Monomerengemisch
33,30% Füller gemäß Beispiel 7
42,34% silanisiertes Siliciumdioxid (AEROSIL® OX-50).

Beim Ermüdungstest hält der Prüfkörper 2000 Zyklen stand. Das Rißbild ist mit dem des Beispiels 13 vergleichbar.

BEISPIEL 15

Eine weitere Paste wird gemäß 13 mit folgender Zusammensetzung hergestellt:
27,5% Monomerengemisch gemäß Beispiel 14
27,5% silanisiertes Siliciumdioxid (AEROSIL® OX-50)
45,0% Füller gemäß Beispiel 3.

Bei einem geradlinigen Rißverlauf durch die Füllerkörner treten die ersten Risse nach 100 Zyklen auf.

### BEISPIEL 16 (Vergleichsbeispiel)

Die Paste wird analog dem Beispiel 14 hergestellt. Anstelle des Füllers des Beispiels 7 wird jedoch der Füller aus Vergleichsbeispiel 12 eingesetzt.

Beim Ermüdungstest treten Risse bereits nach 10 Zyklen auf. Die Risse verlaufen überwiegend entlang der Phasengrenze Füller/Matrix, so daß gezackte Rißlinien entstehen. Dieses Rißbild zeigt deutlich den ungenügenden Verbund zwischen Füller und Matrix.

### BEISPIEL 17

Folgende Pasten werden hergestellt:

|  | Aktivator-Paste | Base-Paste |
|---|---|---|
| – Bis-GMA | 28,96% | 29,09% |
| – Triethylenglycol-dimethacrylat | 10,0% | 10,0% |
| – Benzoylperoxid (BPO 50%ige Paste) | 0,0% | — |
| – N,N-Diethanol-p-toluidin | — | 0,7% |
| – 2,6-Di-tert.-butyl-p-Kresol | 0,04% | 0,01% |
| – übliche UV-Stabilisatoren und optische Aufheller | 0,2% | 0,2% |
| – Siliciumdioxid (AEROSIL® OX-50) | 19,9% | 19,9% |
| – Füller aus Beispiel 2 | 40,0% | 40,0% |
| – Farbpigmente und Titanoxid | 0,1% | 0,1% |
|  | 100,00% | 100,00% |

Die festen Substanzen (BPO, Di-tert.-butyl-p-Kresol, N,N-Diethanol-p-toluidin, UV-Stabilisatoren und optische Aufheller) werden in der jeweiligen Monomerenmischung vollständig gelöst. Die Pasten werden in einer Knetmaschine hergestellt, wobei die Füllstoffe (silanisiertes Siliciumdioxid, Füller aus Beispiel 2) und die Farbpigmente homogen in die entsprechende Monomerlösung eingearbeitet werden.

Die so hergestellten Pasten haben eine angenehme Konsistenz und sind leicht zu mischen. Die Verarbeitungszeit nach Mischbeginn beträgt 2–3 Minuten.

Das Material wird während 1 h bis 37°C polymerisiert. Ansonsten wird die Prüfung wie in Beispiel 14 durchgeführt.

Risse treten nach 1000 Belastungszyklen auf und verlaufen geradlinig um die Eindruckstelle, was von einem guten Verbund zwischen Füller und Matrix zeugt.

| | |
|---|---|
| –2,2,4-Trimethylhexamethylen-bis-(2-carbamoyloxyethyl)-dimethacrylat | 27,95% |
| – Butandiol-1,4-dimethacrylat | 5,0% |
| – Dibenzoylperoxid | 2,0% |
| – Di-tert.-butyl-p-Kresol | 0,05% |
| – Siliciumdioxid (AEROSIL® R972) | 10,0% |
| – Füller aus Beispiel 1 | 10,0% |
| – Farbpigmente | 0,2% |
| | 100,00% |

Die Herstellung der Paste erfolgt wie im Beispiel 17 beschrieben. Es entsteht eine feste, gut verarbeitbare Masse, die für die Herstellung von künstlichen Zähnen als Inlay-Material oder als Verblendkunststoff für Kronen- und Brückenarbeiten geeignet ist.

Die Polymerisation der Prüfkörper für den Ermüdungstest wird in einem Druckpolymerisationsgerät (Ivomat®) bei 120°C und 6 bar während 10 min durchgeführt. Die Prüfung erfolgt wie in Beispiel 13. Nach 100 Belastungszyklen treten erste geradlinige Risse auf.

**Patentansprüche**

1. Reaktiver organischer Füllstoff in Form eines Feststoffpulvers, dadurch gekennzeichnet, daß er durch eine nicht radikalisch ausgelöste Reaktion hergestellt ist und mit Lösungsmitteln nicht extrahierbare, reaktive Doppelbindungen (bestimmt mittels der DSC-Methode) in einer Menge von mindestens 0,5 mMol/g des organischen Füllstoffs aufweist.

2. Füllstoff nach Anspruch 1, dadurch gekennzeichnet, daß er durch Umsetzung von Hydroxygruppen enthaltenden (Meth)acrylaten mit Isocyanaten im Verhältnis von OH-Gruppen zu NCO-Gruppen von etwa 1:1 hergestellt ist, wobei eine oder mehrere der Ausgangsverbindungen mindestens trifunktionell sind.

3. Füllstoff nach Anspruch 2, dadurch gekennzeichnet, daß er unter Verwendung eines Triisocyanats hergestellt ist.

4. Füllstoff nach Anspruch 2, dadurch gekennzeichnet, daß er durch Umsetzung eines (Meth)acrylats eines Tri- oder Polyols mit einem Diisocyanat hergestellt ist.

5. Füllstoff nach Anspruch 3, dadurch gekennzeichnet, daß er unter Anwendung eines stöchiometrischen Unterschusses an Hydroxy(meth)acrylat hergestellt und mittels Wasser und/oder eines aliphatischen Polyols vernetzt ist.

6. Füllstoff nach einem der Ansprüche 2 bis 5, dadurch gekennzeichnet, daß er aus aliphatischen Isocyanaten hergestellt ist.

7. Füllstoff nach Anspruch 1, dadurch gekennzeichnet, daß er durch ionische Copolymerisation von Hydroxygruppen enthaltenden (Meth)acrylaten mit einem Epoxyharz und/oder Trioxan in einem im wesentlichen stöchiometrischen Verhältnis hergestellt ist.

8. Füllstoff nach einem der Ansprüche 1 bis 7, dadurch gekennzeichnet, daß er eine mittlere Teilchengröße von 0,5 bis 100 µm aufweist.

9. Füllstoff nach einem der Ansprüche 1 bis 8, dadurch gekennzeichnet, daß er bezogen auf das Gesamtgewicht bis zu 80 Gew.% an anorganischen und/oder nicht reaktiven organischen Füllern enthält, welche durch Zugabe vor der Polymerisation in den reaktiven organischen Füllstoff inkorporiert sind.

10. Füllstoff nach Anspruche 9, dadurch gekennzeichnet, daß der mit NCO- und/oder Epoxidgruppen reagierende Anteil an Oberflächengruppen des anorganischen Füllers beim Einstellen des stöchiometrischen Verhältnisses der Ausgangsverbindungen mitberücksichtigt ist.

11. Verwendung des Füllstoffes nach den Ansprüchen 1 bis 10 in polymerisierbaren Zusammensetzungen, welche als Bindemittel Monomere mit radikalisch polymerisierbaren, ethylenisch ungesättigten Gruppen enthalten.

12. Verwendung nach Anspruch 11, dadurch gekennzeichnet, daß der Füllstoff radikalisch polymerisierbaren Dentalmaterialien zugesetzt wird, welche als Bindemittel Vinylverbindungen enthalten.

**Claims**

1. Reactive organic filling material in the form of a powdered solid, characterized in that it is produced by a reaction which is not initiated by radicals, and inthat it has reactive double bonds (determined by means of the Differential Scanning Calorimetry [DSC] method), which are not extractable with solvents, in an amount of at lest 0.5 mMol/g of the organic filling material.

2. Filling material according to claim 1, characterized in that it is produced by the reaction of (meth)acrylates containing hydroxy groups with isocyanates in the ratio of OH-groups to NCO-groups of about 1:1, such that one or more of the parent compounds is at least trifunctional.

3. Filling material according to claim 2, characterized in that it is produced using a triisocyanate.

4. Filling material according to claim 2, characterized in that it is produced by the reaction of a (meth)acrylate of a tri- or polyol with a diisocyanate.

5. Filling material according to claim 3, characterized in that it is produced using a stoichiometric deficit of hydroxy(meth)acrylate, and is cross-linked by means of water and/or an aliphatic polyol.

6. Filling material according to one of claims 2 to 5, characterized in that it is produced from aliphatic isocyanates.

7. Filling material according to claim 1, characterized in that it is produced by the ionic copolymerization of (meth)acrylates containing hydroxy groups with an epoxy resin and/or trioxan in an essentially stoichiometric ratio.

8. Filling material according to one of claim 1 to 7, characterized in that it has an average particle size of 0.5 to 100 µm.

9. Filling material according to one of claims 1 to 8, characterized in that it contains up to 80% by weight of inorganic and/or non-reactive organic filling materials, referred to the total weight, which are incorporated into the reactive organic filling material by addition before the polymerization.

10. Filling material according to claim 9, characterized in that the proportion of surface groups of the inorganic filling material which react with NCO- and/or epoxide groups is taken into consideration when adjusting the stoichiometric ratio of the parent compounds.

11. Use of the filling material according to claims 1 to 10 in polymerizable compositions which contain monomers with radical-polymerizable, ethylene-unsaturated groups as the binder.

12. Use according to claim 11, characterized in that the filling material is added to radical-polymerizable dental materials which contain vinyl compounds as the binder.

**Revendications**

1. Charge réactive organique sous forme d'un solide pulvérulent, caractérisée en ce qu'elle est fabriquée par une réaction non-radicalaire et qu'elle renferme au moins 0,5 mMol de doubles liaisons réactives non-extractibles avec des solvants (dosées d'après la technique DSC) par gramme de la charge organique.

2. Charge selon la revendication 1, caractérisée en ce qu'elle est fabriquée par réaction de (méth)acrylates renfermant des groupes hydroxyles avec des isocyanates, selon une proportion groupes OH/groupes NCO d'environ 1:1, au moins un des composés de départ étant au moins trifonctionnel.

3. Charge selon la revendication 2, caractérisée en ce qu'elle est fabriquée par utilisation d'un triisocyanate.

4. Charge selon la revendication 2, caractérisée en ce qu'elle est fabriquée par réaction d'un (méth)acrylate d'un tri- ou polyol avec un diisocyanate.

5. Charge selon la revendication 3, caractérisée en ce qu'elle est fabriquée par la mise en œuvre d'un déficit stoechiométrique d'hydroxy(méth)acrylate et qu'elle est réticulée par l'eau et/ou un polyol aliphatique.

6. Charge selon l'une des revendications 2 à 5, caractérisée en ce qu'elle est fabriquée à partir d'isocyanates aliphatiques.

7. Charge selon la revendication 1, caractérisée en ce qu'elle est fabriquée par copolymérisation ionique de (méth)acrylates renfermant des groupes hydroxyles avec une résine époxy et/ou du trioxanne, selon une proportion pratiquement stoechiométrique.

8. Charge selon l'une des revendications 1 à 7, caractérisée en ce qu'elle présente une taille moyenne des particules de 0,5 à 100 μm.

9. Charge selon l'une des revendications 1 à 8, caractérisée en ce qu'elle renferme jusqu'à 80% en poids (par rapport au poids total) de charges inorganiques et/ou organiques non-réactives, qui sont incorporées par addition, avant polymérisation, à la charge organique réactive.

10. Charge selon la revendication 9, caractérisée en ce que l'on prend en compte, lors de l'établissement des proportions stoechiométriques des composés de départ, les groupes de surface de la charge inorganique qui réagissent avec les groupes NCO et/ou époxydes.

11. Utilisation de la charge selon les revendications 1 à 10 dans des compositions polymérisables, qui renferment, comme liants, des monomères comportant des groupes à insaturation éthylénique polymérisables par un mécanisme radicalaire.

12. Utilisation selon la revendication 11, caractérisée en ce que la charge est ajoutée à des matériaux dentaires polymérisables par voie radicalaire, qui contiennent des composés vinyliques comme liants.